# EUROPEAN PATENT APPLICATION

(11) **EP 2 644 197 A1**
(43) Date of publication of application: **02.10.2013**
(21) Application number: 13160830.9
(22) Date of filing: 25.03.2013
(51) Int. Cl.: A61K 31/522, A61K 9/20

(54) **Novel Pharmaceutical Compositions of Entecavir**

(30) Priority: 26.03.2012 TR 201203388; 06.04.2012 TR 201203956
(71) Applicant: Sanovel Ilac Sanayi ve Ticaret A.S., 34460 Istanbul (TR)
(72) Inventor: Cifter, ÜMIT, 34460 Istanbul (TR); Türkyilmaz, Ali, 34460 Istanbul (TR); Mutlu, Onur, 34460 Istanbul (TR)
(74) Representative: Sevinç, Erkan

(57) **Abstract**

This invention is related to a novel pharmaceutical composition comprising entecavir monohydrate having a median particle size greater than 50 µm and its preparation and use thereof.

## Description

### Field of the Invention

This invention is related to a novel pharmaceutical composition comprising entecavir monohydrate having a median particle size greater than 50 µm and its preparation and use thereof.

### Background of the Invention

Entecavir is a guanosine nucleoside analogue with selective activity against Hepatitis B virus (HBV). The chemical name for entecavir is 2-amino-1,9-dihydro-9-[(1S,3R,4S)-4hydroxy-3-(hydroxymethyl)-2-methylenecyclopentyl]-6H-purin-6-one, monohydrate. Entecavir has the following structural formula shown in below:

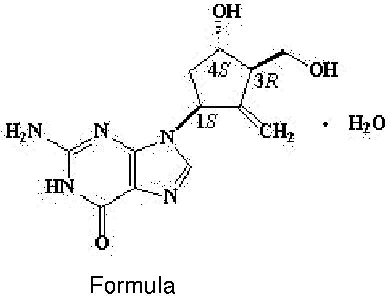

Entecavir is marketed under the name of BARACLUDE, available for oral administration as tablet and oral solutions in strengths of 0.5 mg and 1 mg of entecavir, and it is indicated for the treatment of chronic hepatitis B virus infection in adults.

Entecavir and its use in treating hepatitis B are disclosed in U.S.Patent 5,206,244. Methods for the synthesis of entecavir are disclosed in WO 98/09964.

This invention relates to pharmaceutical compositions for oral administration containing a low dose entecavir. However, it is known that low dose pharmaceutical compositions have the problem of obtaining adequate content uniformity. Because it is difficult to homogenize the low active ingredient in final dosage form and some other difficulties may occur compressing them and this may cause inadequate content uniformity of the final dosage forms. Various processes can be used to get over this problem but they are more complicated and time consuming. Thus, it would be also desirable to provide the solid dosage formulation which can be easily processed without requiring additional expensive and difficult manufacturing technologies.

It is also known in the pharmaceutical field that, when formulating a composition with low dose pharmaceutical active ingredients for administration to those in need of therapy, the challenge is to ensure an even distribution of the pharmaceutically active ingredients throughout the pharmaceutical excipients to ensure a proper dosage and homogeneity. Therefore, it would be desirable to provide solid oral dosage forms that have an adequate content uniformity and that disperse well upon oral administration.

Accordingly, it is known that entecavir has a very low dose in pharmaceutical compositions and have the problem of obtaining adequate content uniformity. Because it is difficult to homogenize in final dosage forms and some other difficulties may occur when compressing them and this may cause inadequate content uniformity and poor dissolution profile of the final dosage forms. To avoid these problems generaly polyvinylpyrrolidone (povidone) and/or cross-lynked polyvinylpyrrolidone (crospovidone) is used to obtain a good dispersion, but this is not enough alone and particle size reduction of the active ingredient is also preffered.

However, particle size reduction is not always effective enough for obtaining adequate content uniformity and for increasing the dissolution rate of a drug to a certain required value. Many drugs having low dose such as from 0.01 to 2.0 mg, have a strong tendency to agglomerate during the manufacturing process into larger particles with an overall decrease in effective surface area. The technical problem of particle size reduction may occur during the manufacturing and tabletting procedure such as agglomeration and bad flowabilty because of having less glidant and lubricant characteristics of powders or granules. Further, flow properties of drugs can be influenced by particle size, and particle size reduction to extremely small sizes (such as less than 10 µm) may be inadvisable for some drug substances. Such effects act as dissolution rate limiting steps since they minimize maximum drug surface-liquid contact.

It is also known that, using large particle size has also disadvantages over flow behaviour and homogeneity and thus processability of the powder having large particles becomes difficult.

Thus, there is need in the art for pharmaceutical compositions of entecavir for oral administration, which has an adequate content uniformity causing a good dispersion upon oral administration wich is easily processed and has high bioavailability and improved stability for their preparation and use thereof.

### Summary of the Invention

The present invention provides a novel pharmaceutical composition comprising entecavir monohydrate having a median particle size (d₅₀) greater than 50 µm and at least one pharmaceutically acceptable excipient which overcomes the above described problems in prior art and have additive advantages over them.

The main object of the present invention is to obtain adequate content uniformity of low dosage forms of entecavir monohydrate with using adequate excipients having a median particle size also greater than 50 µm and improved processes.

There is also provided an improved process for preparing the entecavir which has a low dose active ingredient. It is difficult to prepare entecavir formulations with a simple process such as direct compression because of having low doses of entecavir such as from 0.01 to 2.0 mg.

Another object of this invention is to provide an improved stability with high bioavailability of pharmaceutical compositions of entecavir monohydrate.

One of the preffered embodiments of this invention is directed to narrow particle size distrubition of entecavir composition wherein the ratio between the median particle size (d₅₀) and d₉₀ is equal to or greater than 0.40. Preferably, the ratio between the median particle size and d d₉₀ is between 0.45 and 0.50, more preferably it is 0.50 and 0.70.

A further embodiment of the invention is the pharmaceutical composition comprising entecavir monohydrate having a median particle size greater than, 75 µm; preferably having a median particle size greater than 100 µm.

A further embodiment of the invention is the pharmaceutical composition comprising entecavir monohydrate having a median particle size between 100 and 500 µm; preferably having a median particle size between 100 and 300 µm.

A further embodiment of the invention is the pharmaceutical composition comprising pharmaceutically acceptable excipients having a narrow particle size distrubition wherein the ratio between d₅₀ and d₉₀ is equal to or greater than 0.40.

As a further embodiment, pharmaceutically acceptable excipient is sodium starch glycolate.

As a further embodiment, pharmaceutically acceptable excipient is sodium stearyl fumarate.

As a further embodiment, pharmaceutically acceptable excipient is hydroxypropyl methylcellulose.

According to another embodiment of the invention, the pharmaceutical composition doesn't comprise povidone and/or crospovidone.

Yet, according to another embodiment, the invention provides the pharmaceutical composition of entecavir monohydrate comprising;
a. 0.01 - 5.0 % of entecavir monohydrate
b. 1.0 - 30 % of starch
c. 1.0 - 30 % of sodium starch glycolate
d. 10.0 - 80 % of microcrystalline cellulose
e. 5.0 - 30 % of mannitol
f. 0.10 - 10.0 % of hydroxypropyl methyl cellulose
g. 0.10 - 5.0 % of sodium stearyl fumarate
h. 0.01 - 2.0 % of colloidal silicon dioxide

As a further embodiment, the pharmaceutical composition is in the form of tablets, capsules, powders, sachets; preferably the final dosage form is in the form of a tablet.

As a further embodiment, the invention provides the final dosage form which has a content uniformity of less than 2.0% RSD, preferably less than 1.0 % RSD.

As a further embodiment, the invention provides a direct compression process for preparing the pharmaceutical composition comprising the following steps;
a. blending entecavir monohydrate, microcrystalline cellulose, mannitol, sodium starch glycoloate and hydroxypropylmethylcellulose progressively, wherein the blending time is preferably 20 min.,
b. adding sodium stearyl fumarate to the powder mixture above and blending progressively for about 5 min.
c. compressing the final powder mixture to form tablets,
d. coating the tablets

As a further embodiment, the invention provides a granulation process for preparing the pharmaceutical composition comprising the following steps;
a. blending entecavir monohydrate, 1/3 of microcrystalline cellulose and hydroxypropylmethylcelluloses progressively,
b. compacting the blended mixture,
c. adding mannitol, 80% of colloidal silicon dioxide, 2/3 of microcrystalline cellulose and sodium starch glycoloate to this mixture of step b., and further progressive blending for 15 min.,
d. adding sodium stearyl fumarate and rest of the colloidal silicon dioxide and blending this mixture until obtaining a homogenous powder mixture,
e. compressing the blended mixture to form tablets
f. coating the tablets

As a further embodiment, the invention provides a wet granulation process for preparing the pharmaceutical composition comprising the following steps;
a. adding hydroxypropyl methylcellulose, starch and 20% by weight of mannitol and entecavir in a granulator and blending them for 10 min.
b. adding water to this mixture and blending to form granules
c. sieving and drying the wet granules,
d. adding microcrystalline cellulose and rest of the mannitol and blending them for 10 min.
e. adding colloidal silicon dioxide and sodium stearyl fumarate to this mixture and blending them until obtaining a homogenous powder mixture,
f. compressing the blended mixture to form tablets
g. coating the tabets

Yet, another embodiment of the invention is the pharmaceutical composition for use in the treatment of hepatitis B virus infection.

Further advantages and embodiments of the present invention will become apparent from the following description

### Detailed Description of the Invention

As used here in, "particle size distribution" is defined by the cumulative volume size distrubition as tested by a conventionally accepted method which is the laser diffraction method determined by the equipment of Malvern Mastersizer 2000. "median particle size" is defined by "d₅₀", the size at which 50% by volume of the particles are finer and "d₉₀" means that the size at which %90 by volume of the particles are finer. "narrow particle size distribution" is defined by the ratio between the median particle size (d₅₀) and the particle size at d₉₀.

It has been found that contrary to prior art and the general knowledge of the skilled person in the art entecavir having a particle size greater than 50 micron or more such as 100 micron does not show the expected disadvantages in flow behaviour and homogeneity and thus processabilty if it is provided as particles having a narrow particle size distribution. In this case the active ingredient can be easly handled, mixed with excipients even having a larger particle size and can be processed into pharmaceutical compositions such as tablets or capsules without the known and expected problems such as segregation or de-mix during the process steps.

In a further embodiment, the pharmaceutical composition of this invention comprises entecavir having a median particle size (d₅₀) greater than 50 µm, preferably greater than 75 µm, more preferably greater than 100 µm and having particle size of d₉₀ greater than 100 µm, preferably greater than 150 µm.

In order to achieve the unexpected good results of the present invention the entecavir must have a narrow particle size distribution. According to this embodiment, this ratio of d₅₀ to d₉₀ must be equal to or greater than 0.40, preferably greater than 0.45, more preferably greater than 0.50 and most preferably between 0.50 and 0.70. Thus, an adequate content uniformity is achieved in the final dosage forms, wherein the content uniformity is less than 2.0 % RSD (Relative Standard Deviation), preferably it is less than 1.0 % RSD. Herein, content uniformity test is determined by calculating RSD (Relative Standard Deviation) value of 10 tablets (According to European Pharmacopea 7.0, page.315-317, 2.9.40. Uniformity Of Dosage Units).

As a further embodiment, the friability is less than 1.0 %.

Accordingly the pharmaceutical acceptable excipients also have a narrow particle size distribution wherein the ratio is equal to or greater than 0.40, preferably greater than 0.45, more preferably greater than 0.50 and most preferably between 0.50 and 0.70. This helps to improve the dissolution profile and to obtain adequate content uniformity without causing the disadvantages of having larger particle size especially when the particle size is greater than 50 micron, or even more such as greater than 100 micron.

Accordingly, we have found that, solid oral dosage forms such as tablets, when prepared with sodium starch glycolate have good storage properties. Sodium starch glycolate has advantages over other unmodified starches because unmodified starch has poor flow characteristics and tends to increase tablet friability and capping if used in high concentrations. Additionally, the improved flow and lubricity characteristics of sodium starch glycolate can impart further benefit to the formulation in a very cost-effective manner when if used having a median particle size greater than 50 µm.

It is also observed that, sodium stearyl fumarate is less sensitive to overblending and compressibility than other lubricants, this gives the advantage to the present formulations to have better compressibility and robustness to overblending without causing poor dissolution profiles especially when used having a median particle size greater than 50 µm.

Accordingly it is also found that when sodium starch glycolate and sodium stearyl fumarate is used with entecavir monohydrate, the pharmaceutical compositions of this invention has better storage stability and better compressibility with less friability.

According to another object of the present invention, the pharmaceutical composition of entecavir monohydrate further comprising hydroxyl propyl methyl cellulose (HPMC) having a median particle size greater than 50 µm. Accordingly, a synergistic effect is observed over the distribution of the low doses of entecavir monohydrate throughout other pharmaceutical excipients when HPMC is used in a specific weight ratio with entecavir which is 1:5 to 50:1 (w/w), preferably the ratio is 1:2 to 25:1 (w/w), more preferably the ratio is 1:1 to 10:1 (w/w).

Yet another embodiment of the invention is the pharmaceutical composition which doesn't comprise povidone and/or crospovidone. Using them, may cause stability problems because of their hygroscopic properties and they are more hygroscopic other than the other disintegrants especially when used in high amounts to obtain a good dispersion for the cotent uniformity of the final dosage form. Also because of binding properties of povidone the dissolution may be effected in negative way.

Accordingly, it is even possible to prepare tablets by direct compression without the requirement of previous granulation of the active ingredient into larger particles in order to increase the flow properties. Likewise the granulation is possible as well, because the flow behaviour of the entecavir of a particle size greater than 50 micron or more such as 100 micron, and a narrow particle size distribution facilitates the granulation process.

Thus, it is possible to prepare pharmaceutical compositions with the entecavir of the present invention in a fast and cost efficient manner.

Accordingly a progressive blending technique is used to improve the distribution of the low dose active ingredient, entecavir. Progressive blending is a technique used in mixing two excipients of unequal quantities, where one begins with the smallest quantity and adds an equal quantity of the excipient having the larger amount; process continues until all of the ingredients are used. Also, when the formulation is prepared by the excipients described above, improves the formulation to an easily processed formulation.

The preferred direct compression process of the present invention for preparing the pharmaceutical composition of entecavir monohydrate comprises the following steps;
e. blending entecavir monohydrate, microcrystalline cellulose, mannitol, sodium starch glycoloate and hydroxypropylmethylcellulose progressively, wherein the blending time is preferably 20 min.,
f. adding sodium stearyl fumarate to the powder mixture above and blending progressively for about 5 min.
g. compressing the final powder mixture to form tablets,
h. coating the tablets.

The preferred granulation process of the present invention for preparing the pharmaceutical composition of entecavir monohydrate comprises the following steps;
g. blending entecavir monohydrate, 1/3 of microcrystalline cellulose and hydroxypropylmethylcelluloses progressively,
h. compacting the blended mixture,
i. adding mannitol, 80% of colloidal silicon dioxide, 2/3 of microcrystalline cellulose and sodium starch glycoloate to this mixture of step b., and further progressive blending for 15 min.,
j. adding sodium stearyl fumarate and rest of the colloidal silicon dioxide and blending this mixture until obtaining a homogenous powder mixture,
k. compressing the blended mixture to form tablets
l. coating the tablets.

The preffered wet granulation process for preparing the pharmaceutical composition of entecavir monohydrate comprising the following steps;
a. adding hydroxypropyl methylcellulose, starch and 20% by weight of mannitol and entecavir in a granulator and blending them for 10 min.
b. adding water to this mixture and blending to form granules,
c. sieving and drying the wet granules,
d. adding microcrystalline cellulose and rest of the mannitol and blending them for 10 min.
e. adding colloidal silicon dioxide and sodium stearyl fumarate to this mixture and blending them until obtaining a homogenous powder mixture,
f. compressing the blended mixture to form tablets
g. coating the tabets.

One of the preffered embodiments of this invention is using high shear granulator during wet granulation process instead of fluid bed dryer to get over some technical problems such as described below;
a. to obtain a good flowability and compressibility of the final blended powder mixture,
b. to obtain hard granules at the end of the process which lessens the dustiness, and this provides less friable granules,
c. narrowing the particule size distribution of the powder mixtures,
d. to ensure an even distribution of the pharmaceutically active ingredient throughout the pharmaceutical excipients to ensure a proper dosage and homogeneity and this has an additive effect over content uniformity,
e. using less binder solution and this makes the timing of the process shorter,
f. to improve dissolution characteristics of the final dosage forms.

As a further embodiment, the pharmaceutical compositions according to the present invention may further comprise one or more other active ingredients such as tenofovir.

In a further embodiment, the pharmaceutical compositions of this invention are suitable for the treatment of hepatit - B and complications or disorders associated therewith.

As a further embodiment, the pharmaceutical compositions of entecavir monohydrate comprise at least one pharmaceutically acceptable excipient. Suitable pharmaceutically acceptable excipients comprise but are not limited to disintegrants, fillers, binders, lubricants and glidants.

Suitable disintegrants other than sodium starch glycolate, may comprise but not limited to croscarmellose sodium, alginic acid, sodium alginate, microcrystalline cellulose, carboxymethylcellulose sodium, docusate sodium, low-substituted hydroxypropyl cellulose, magnesium aluminum silicate, poloxamer, sodium glycine carbonate and mixtures thereof.

Suitable fillers may comprise but not limited to starch, microcrystalline cellulose, mannitol, lactose, dibasic calcium phosphate dihydrate, polysaccharides, sugars, sorbitol, mannitol, maltitol, sucrose, maltodextrin, sucrose-maltodextrin coprecipitate, Xylitol and mixtures thereof. The most preffered fillers are starch, microcrystalline cellulose and mannitol.

Suitable binders other than hydroxyl propyl methyl cellulose, may comprise but not limited to hydroxypropyl cellulose, carboxy methyl cellulose, methyl cellulose, gelatin, natural gums, polymethycrylate and mixtures thereof.

Suitable lubricants other than sodium stearyl fumarate may comprise but not limited to polyethylene glycol, stearic acid, metal stearates, talc, boric acid, sodium chloride benzoate and acetate, sodium or magnesium lauryl sulfate and mixtures thereof.

Suitable glidants may comprise but not limited to colloidal silicon dioxide, talc, aluminium silicate, calcium silicate, magnesium silicate, magnesium carbonate, magnesium oxide and mixtures thereof. The most preffered glidant is colloidal silicon dioxide.

This invention is further defined by reference to the following examples. Although the examples are not intended to limit the scope of the present invention, they should be considered in the light of the description detailed above.

### Example 1 : Entecavir monohydrate tablet formulation

| **Ingredients** having d₅₀ greater than 50µm | **Amount mg** |
|---|---|
| entecavir monohydrate | 1.00 |
| starch | 60.0 |
| sodium starch glycolate | 60.0 |
| Microcrystalline cellulose (pH-102) | 180.0 |
| Mannitol | 70.0 |
| Hydroxypropyl methylcellulose | 10.0 |
| Sodium stearyl fumarate | 12.0 |
| Colloidal silicon dioxide | 2.0 |
| **Total tablet weight** | **395.0** |

| | |
|---|---|
| "d₅₀" (median particle size) which is greater than 50µm, is measured by the laser diffraction method determined by the equipment of Malvern Mastersizer 2000, three measurement per aliquot in 60 s, intervals. | |

The formulations of these examples can be manufactured according to the processes described detailed above in the description and tablet formulations further comprise a film coating in an amount of 2.5 % which is disclosed in detail below.

### Coating with:

| | |
|---|---|
| HPMC - Methocel | 62,50 % |
| Titanium dioxide | 28.75 % |
| Poylethyleneglycol 400 | 6.25 % |
| FD&C yellow & sunset yellow | 2.50 % |

### Example 2:

The pharmaceutical composition of this present invention (Ex.1) was tested for its dissolution profile against an entecavir composition including povidone and crospovidone instead of sodium starch glycolate and sodium stearyl fumarate in 1000 ml of 0.05 M phosphate buffer at pH 6.8, at 37ºC using a USP paddle II method rotating at 50 RPM. According to the results the entecavir formulation of the present invention (Ex.1) has a high dissolution profile at least 85% in 15 min., while the comperative formulation has a poor dissolution profile lower than 85% in 15 min. Thus, the above mentioned excipients, especially sodium starch glycolate and sodium stearyl fumarate increase the solubility of entecavir.

### Example 3:

The pharmaceutical composition of this present invention (Ex.1) was tested for its content uniformity against the same comperative entecavir formulation used in Ex.2. According to the results the present formulation of Ex.1 has a content uniformity which is less than 0.50% RSD while the comperative formulation has a content uniformity higher than 0.50% RSD even 1.0 % RSD. Herein, content uniformity test is determined by calculating RSD (Relative Standard Deviation) value of 10 tablets.

### Referance Product as comperative formulation used in Ex 2 and 3:

Entecavir monohydrate (having d₅₀ less than 50 µm) as active ingredient and lactose monohydrate, crospovidone, povidone, microcrystalline cellulose, colloidal silicon dioxide, magnesium stearate as inactive ingredients.

## Claims

1. A pharmaceutical composition comprising entecavir monohydrate having a median particle size (d₅₀) greater than 50 µm and at least one pharmaceutically acceptable excipient.

2. The pharmaceutical composition according to claim 1, comprising entecavir monohydrate having a median particle size greater than 75 µm.

3. The pharmaceutical composition according to claim 2, comprising entecavir monohydrate having a median particle size greater than 100 µm.

4. The pharmaceutical composition according to claim 3, comprising entecavir monohydrate having a median particle size between 100 and 500 µm.

5. The pharmaceutical composition according to claim 4, comprising entecavir monohydrate having a median particle size between 100 and 300 µm.

6. The pharmaceutical composition according to claims 1 to 4, wherein entecavir monohydrate has a particle size ratio between d₅₀ and d₉₀ which is equal to or greater than 0.40.

7. The pharmaceutical composition according to claim 6, wherein entecavir monohydrate has a particle size ratio between d₅₀ and d₉₀ which is equal to or greater than 0.45, preferably greater than 0.50.

8. The pharmaceutical composition according to claim 7, wherein entecavir monohydrate has a particle size ratio of d₅₀ to d₉₀ which is between 0.50 and 0.70.

9. The pharmaceutical composition according to claim 1, wherein the pharmaceutically acceptable excipients have a particle size ratio between d₅₀ and d₉₀ which is equal to or greater than 0.40.

10. The pharmaceutical composition according to claim 9, wherein the pharmaceutically acceptable excipient is sodium starch glycolate.

11. The pharmaceutical composition according to claim 9, wherein the pharmaceutically acceptable excipient is sodium stearyl fumarate.

12. The pharmaceutical composition according to claim 9, wherein the pharmaceutically acceptable excipient is hydroxypropyl methylcellulose.

13. The pharmaceutical composition according to any preceding claims, wherein said composition doesn't comprise povidone and/or crospovidone.

14. The pharmaceutical composition according to any preceding claims, comprising;
a. 0.01 - 5.0 % of entecavir monohydrate,
b. 1.0 - 30 % of starch,
c. 1.0 - 30 % of sodium starch glycolate,
d. 10.0 - 80 % of microcrystalline cellulose,
e. 5.0 - 30 % of mannitol,
f. 0.10 - 10.0 % of hydroxypropyl methyl cellulose,
g. 0.10 - 5.0 % of sodium stearyl fumarate,
h. 0.01 - 2.0 % of colloidal silicon dioxide.

15. The pharmaceutical composition according to any preceding claims, wherein the pharmaceutical composition is in the form of tablets, capsules, powders, sachets; preferably the final dosage form is in the form of a tablet.

16. The pharmaceutical composition according to claims 14 and 15, wherein the final dosage form has a content uniformity of less than 2.0% RSD , preferably less than 1.0 % RSD.

17. A direct compression process for preparing the pharmaceutical composition according to any preceding claims, comprising the following steps;
a. blending entecavir monohydrate, microcrystalline cellulose, mannitol, sodium starch glycoloate and hydroxypropylmethylcellulose progressively, wherein the blending time is preferably 20 min.,
b. adding sodium stearyl fumarate to the powder mixture above and blending progressively for about 5 min.
c. compressing the final powder mixture to form tablets,
d. coating the tablets

18. A granulation process for preparing the pharmaceutical composition according to any preceding claims, comprising the following steps;
a. blending entecavir monohydrate, 1/3 of microcrystalline cellulose and hydroxypropylmethylcelluloses progressively,
b. compacting the blended mixture,
c. adding mannitol, 80% of colloidal silicon dioxide, 2/3 of microcrystalline cellulose and sodium starch glycoloate to this mixture of step b., and further progressive blending for 15 min.,
d. adding sodium stearyl fumarate and rest of the colloidal silicon dioxide and blending this mixture until obtaining a homogenous powder mixture,
e. compressing the blended mixture to form tablets
f. coating the tablets

19. A wet granulation process for preparing the pharmaceutical composition according to any preceding claims, comprising the following steps;
a. adding hydroxypropyl methylcellulose, starch and 20% by weight of mannitol and entecavir in a granulator and blending them for 10 min.
b. adding water to this mixture and blending to form granules
c. sieving and drying the wet granules,
d. adding microcrystalline cellulose and rest of the mannitol and blending them for 10 min.
e. adding colloidal silicon dioxide and sodium stearyl fumarate to this mixture and blending them until obtaining a homogenous powder mixture,
f. compressing the blended mixture to form tablets
g. coating the tablets

20. The pharmaceutical composition according to any preceding claims, for use in the treatment of hepatitis B virus infection.
